# FASCICULE DE BREVET EUROPEEN

(11) **EP 1 781 691 B1**
(45) Date de publication et mention de la délivrance du brevet: **16.12.2009**
(21) Numéro de dépôt: 05778855.6
(22) Date de dépôt: 21.06.2005
(51) Int. Cl.: C07K 14/195

(54) **NOUVELLES PROTEINES PERMETTANT, NOTAMMENT, LA MISE EN EVIDENCE IN VITRO ET LA PREVENTION DES INFECTIONS A LEGIONELLA PNEUMOPHILA**
NEUE PROTEINE, Z.B. ZUR VERWENDUNG FÜR DIE IN-VITRO-ISOLIERUNG UND PRÄVENTION VON LEGIONELLA PNEUMOPHILA-INFEKTIONEN
NOVEL PROTEINS WHICH CAN BE USED, FOR EXAMPLE, FOR THE IN VITRO ISOLATION AND PREVENTION OF LEGIONELLA PNEUMOPHILA INFECTIONS

(30) Priorité: 06.08.2004 FR 0408714
(43) Date de publication de la demande: 09.05.2007
(73) Titulaire: InGen Biosciences, 91380 Chilly-Mazarin (FR); UNIVERSITE CLAUDE BERNARD - LYON 1, 69100 Villeurbanne (FR)
(72) Inventeur: ARIE, Jean-Philippe, F-33000 Bordeaux (FR); CYNCYNATUS, Camille, F-94410 Saint-Maurice (FR)
(74) Mandataire: Jacobson, Claude
(86) Numéro de dépôt international: PCT/FR2005/001549
(87) Numéro de publication internationale: WO 2006/027431

(56) Documents cités:
- HELBIG JÜRGEN H ET AL: "Clinical utility of urinary antigen detection for diagnosis of community-acquired, travel-associated, and nosocomial legionnaires' disease." JOURNAL OF CLINICAL MICROBIOLOGY. FEB 2003, vol. 41, no. 2, février 2003 (2003-02), pages 838-840, XP002313797 ISSN: 0095-1137
- CHIEN MINCHEN ET AL: "The genomic sequence of the accidental pathogen Legionella pneumophila." SCIENCE. 24 SEP 2004, vol. 305, no. 5692, 24 septembre 2004 (2004-09-24), pages 1966-1968, XP002313850 ISSN: 1095-9203
- CAZALET CHRISTEL ET AL: "Evidence in the Legionella pneumophila genome for exploitation of host cell functions and high genome plasticity." NATURE GENETICS. NOV 2004, vol. 36, no. 11, novembre 2004 (2004-11), pages 1165-1173, XP002313851 ISSN: 1061-4036

### LISTAGE DE SEQUENCES

<110> ABAG et Université Claude Bernard Lyon 1
<120> Nouvelles protéines permettant, notamment, la mise en évidence in vitro et la prévention des infections à Legionella pneumophila
<130> B3895
<150> FR 04 08714
   <151> 2004-08-06
<160> 2
<170> PatentIn version 3.1
<210> 1
   <211> 1332
   <212> DNA
   <213> Legionella pneumophila
<220>
   <221> CDS
   <222> (1)..(1332)
   <223>
<400> 1
   acg ttt gtt tta aag gaa ttt gat gca cta aaa agt cat ttt aac gac       48
   Thr Phe Val Leu Lys Glu Phe Asp Ala Leu Lys Ser His Phe Asn Asp
   1               5                   10                  15
   acc gtt aaa atc atc ctt caa cgc gaa aaa aag gac aaa att gaa gac       96
   Thr Val Lys Ile Ile Leu Gln Arg Glu Lys Lys Asp Lys Ile Glu Asp
               20                  25                  30
   ctt ccc aac ccc aga aaa gaa gag ctt caa ttt ctg acc gct gtt ctc      144
   Leu Pro Asn Pro Arg Lys Glu Glu Leu Gln Phe Leu Thr Ala Val Leu
           35                  40                  45
   aat caa ttg gaa gca aaa atc gat gaa ctg aaa cca cgt tct ttg gcc      192
   Asn Gln Leu Glu Ala Lys Ile Asp Glu Leu Lys Pro Arg Ser Leu Ala
       50                  55                  60
   agc tat gtc cat gta ttt tat ggt gct atg ctg ctt gtc tgt aaa gac      240
   Ser Tyr Val His Val Phe Tyr Gly Ala Met Leu Leu Val Cys Lys Asp
   65                  70                  75                  80
   gtt gaa aat aat ctg cgg gtg atg gaa aag aaa gaa aac agc ttg ctg      288
   Val Glu Asn Asn Leu Arg Val Met Glu Lys Lys Glu Asn Ser Leu Leu
                   85                  90                  95
   ttt act cgc tta atg gat ggt atg ggt att tct gat gaa aat ata cca      336
   Phe Thr Arg Leu Met Asp Gly Met Gly Ile Ser Asp Glu Asn Ile Pro
               100                 105                 110
   aca tct gag cag aac atc atg ttt tac aga gga tta aac aaa ttc tta      384
   Thr Ser Glu Gln Asn Ile Met Phe Tyr Arg Gly Leu Asn Lys Phe Leu
           115                 120                 125
   aat ttc att tat gaa agc aat gat tct cgt aaa ggc tta aaa aag gag      432
   Asn Phe Ile Tyr Glu Ser Asn Asp Ser Arg Lys Gly Leu Lys Lys Glu
       130                 135                 140
   cat ttc ctg caa gtc ctt tcg tta aaa aag ata tac tct tta gcc aaa      480
   His Phe Leu Gln Val Leu Ser Leu Lys Lys Ile Tyr Ser Leu Ala Lys
   145                 150                 155                 160
   tta agt tat gag cag gaa gag gca gct gaa aat aat gct ttg gca aaa      528
   Leu Ser Tyr Glu Gln Glu Glu Ala Ala Glu Asn Asn Ala Leu Ala Lys
                   165                 170                 175
   tta act gct gat gga aaa acc aaa gcc aat gcg aac agc ttc cat gtg      576
   Leu Thr Ala Asp Gly Lys Thr Lys Ala Asn Ala Asn Ser Phe His Val
               180                 185                 190
   gaa aaa cca atc gat tca tcc att gtt gag caa ttc aaa tcc tgg gat      624
   Glu Lys Pro Ile Asp Ser Ser Ile Val Glu Gln Phe Lys Ser Trp Asp
           195                 200                 205
   gaa atg aaa ggc gct ctt cat caa tta att ctc gat gaa ctt tct gat      672
   Glu Met Lys Gly Ala Leu His Gln Leu Ile Leu Asp Glu Leu Ser Asp
       210                 215                 220
   aag aac gtt gct aaa att tca gct tta agt caa gcc cgc tcc gca caa      720
   Lys Asn Val Ala Lys Ile Ser Ala Leu Ser Gln Ala Arg Ser Ala Gln
   225                 230                 235                 240
   ctg aaa ttt tta caa acg atg gca gaa caa cta gac aag atc cct aac      768
   Leu Lys Phe Leu Gln Thr Met Ala Glu Gln Leu Asp Lys Ile Pro Asn
                   245                 250                 255
   caa tct ctt gag ccg tct gag aaa atg gcc att ctt gct gga gca atg      816
   Gln Ser Leu Glu Pro Ser Glu Lys Met Ala Ile Leu Ala Gly Ala Met
               260                 265                 270
   tat atc gtt cgt ggc caa ata gcc cag gag tat gga aaa gat cca tta      864
   Tyr Ile Val Arg Gly Gln Ile Ala Gln Glu Tyr Gly Lys Asp Pro Leu
           275                 280                 285
   agc aac gat aaa atc agc gct act gtg att cat aca ggt tta agc acg      912
   Ser Asn Asp Lys Ile Ser Ala Thr Val Ile His Thr Gly Leu Ser Thr
       290                 295                 300
   ata ctt cat gcc aat gct gat tgc tgt gaa gac aag gaa gta ctg att      960
   Ile Leu His Ala Asn Ala Asp Cys Cys Glu Asp Lys Glu Val Leu Ile
   305                 310                 315                 320
   gct gct gcg aat aaa ttt att cgt cat atg gtt att gag cgt cct gaa     1008
   Ala Ala Ala Asn Lys Phe Ile Arg His Met Val Ile Glu Arg Pro Glu
                   325                 330                 335
   caa tca aat aaa aaa atc act aag gaa tcc gtt cga gaa aac aac atg     1056
   Gln Ser Asn Lys Lys Ile Thr Lys Glu Ser Val Arg Glu Asn Asn Met
               340                 345                 350
   ttt tcc gac atc gcc ggc ttc caa ttg atc tct gtc ttg acg tta ata     1104
   Phe Ser Asp Ile Ala Gly Phe Gln Leu Ile Ser Val Leu Thr Leu Ile
           355                 360                 365
   caa aac atg atc aaa aca tgt cgt act gat gcc att gaa gct tgt gtc     1152
   Gln Asn Met Ile Lys Thr Cys Arg Thr Asp Ala Ile Glu Ala Cys Val
       370                 375                 380
   acc aag cgt aag gaa gaa ctc gaa gca tta aaa ccc aaa aaa gag ggt     1200
   Thr Lys Arg Lys Glu Glu Leu Glu Ala Leu Lys Pro Lys Lys Glu Gly
   385                 390                 395                 400
   tat tcc att gcg agt tca gtc act gga tat gta ggc agc tgg ttt aaa     1248
   Tyr Ser Ile Ala Ser Ser Val Thr Gly Tyr Val Gly Ser Trp Phe Lys
                   405                 410                 415
   aaa gca cca agc atg tct gaa gaa gac gaa gaa gat gac tta aaa gat     1296
   Lys Ala Pro Ser Met Ser Glu Glu Asp Glu Glu Asp Asp Leu Lys Asp
               420                 425                 430
   caa aac aca gca gaa gag acc agc aaa ccg acc gta                     1332
   Gln Asn Thr Ala Glu Glu Thr Ser Lys Pro Thr Val
           435                 440
<210> 2
   <211> 444
   <212> PRT
   <213> Legionella pneumophila
<400> 2
   Thr Phe Val Leu Lys Glu Phe Asp Ala Leu Lys Ser His Phe Asn Asp
   1               5                   10                  15
   Thr Val Lys Ile Ile Leu Gln Arg Glu Lys Lys Asp Lys Ile Glu Asp
               20                  25                  30
   Leu Pro Asn Pro Arg Lys Glu Glu Leu Gln Phe Leu Thr Ala Val Leu
           35                  40                  45
   Asn Gln Leu Glu Ala Lys Ile Asp Glu Leu Lys Pro Arg Ser Leu Ala
       50                  55                  60
   Ser Tyr Val His Val Phe Tyr Gly Ala Met Leu Leu Val Cys Lys Asp
   65                  70                  75                  80
   Val Glu Asn Asn Leu Arg Val Met Glu Lys Lys Glu Asn Ser Leu Leu
                   85                  90                  95
   Phe Thr Arg Leu Met Asp Gly Met Gly Ile Ser Asp Glu Asn Ile Pro
               100                 105                 110
   Thr Ser Glu Gln Asn Ile Met Phe Tyr Arg Gly Leu Asn Lys Phe Leu
           115                 120                 125
   Asn Phe Ile Tyr Glu Ser Asn Asp Ser Arg Lys Gly Leu Lys Lys Glu
       130                 135                 140
   His Phe Leu Gln Val Leu Ser Leu Lys Lys Ile Tyr Ser Leu Ala Lys
   145                 150                 155                 160
   Leu Ser Tyr Glu Gln Glu Glu Ala Ala Glu Asn Asn Ala Leu Ala Lys
                   165                 170                 175
   Leu Thr Ala Asp Gly Lys Thr Lys Ala Asn Ala Asn Ser Phe His Val
               180                 185                 190
   Glu Lys Pro Ile Asp Ser Ser Ile Val Glu Gln Phe Lys Ser Trp Asp
           195                 200                 205
   Glu Met Lys Gly Ala Leu His Gln Leu Ile Leu Asp Glu Leu Ser Asp
       210                 215                 220
   Lys Asn Val Ala Lys Ile Ser Ala Leu Ser Gln Ala Arg Ser Ala Gln
   225                 230                 235                 240
   Leu Lys Phe Leu Gln Thr Met Ala Glu Gln Leu Asp Lys Ile Pro Asn
                   245                 250                 255
   Gln Ser Leu Glu Pro Ser Glu Lys Met Ala Ile Leu Ala Gly Ala Met
               260                 265                 270
   Tyr Ile Val Arg Gly Gln Ile Ala Gln Glu Tyr Gly Lys Asp Pro Leu
           275                 280                 285
   Ser Asn Asp Lys Ile Ser Ala Thr Val Ile His Thr Gly Leu Ser Thr
       290                 295                 300
   Ile Leu His Ala Asn Ala Asp Cys Cys Glu Asp Lys Glu Val Leu Ile
   305                 310                 315                 320
   Ala Ala Ala Asn Lys Phe Ile Arg His Met Val Ile Glu Arg Pro Glu
                   325                 330                 335
   Gln Ser Asn Lys Lys Ile Thr Lys Glu Ser Val Arg Glu Asn Asn Met
               340                 345                 350
   Phe Ser Asp Ile Ala Gly Phe Gln Leu Ile Ser Val Leu Thr Leu Ile
           355                 360                 365
   Gln Asn Met Ile Lys Thr Cys Arg Thr Asp Ala Ile Glu Ala Cys Val
       370                 375                 380
   Thr Lys Arg Lys Glu Glu Leu Glu Ala Leu Lys Pro Lys Lys Glu Gly
   385                 390                 395                 400
   Tyr Ser Ile Ala Ser Ser Val Thr Gly Tyr Val Gly Ser Trp Phe Lys
                   405                 410                 415
   Lys Ala Pro Ser Met Ser Glu Glu Asp Glu Glu Asp Asp Leu Lys Asp
               420                 425                 430
   Gln Asn Thr Ala Glu Glu Thr Ser Lys Pro Thr Val
           435                 440

## Description

Les légionelloses sont des infections respiratoires sévères résultant de l'inhalation d'aérosols contaminés par des bactéries à croissance intracellulaire du genre Legionella, et qui se manifestent sous forme sporadique ou épidémique. Ces infections surviennent aussi bien en ville (infections communautaires) qu'en milieu hospitalier (infections nosocomiales). *Legionella pneumophila* est responsable de 95% de l'ensemble des cas de légionellose.

Les légionelloses se présentent principalement comme des infections pulmonaires aiguës. Elles se caractérisent par la sévérité du tableau clinique : après une incubation de 2 à 10 jours et un début pseudo-grippal, le malade présente une température élevée, un malaise général, des douleurs abdominales, voire des troubles psychiques. L'atteinte radiologique est souvent bilatérale. La mortalité associée, particulièrement élevée chez certaines personnes dites à risque (sujets âgés ou souffrant d'un cancer, d'une hémopathie), varie de 10 à 20% (13% des 835 cas recensés en France en 2002).

Il est essentiel de pouvoir poser précocement le diagnostic de légionellose du fait de l'enjeu thérapeutique : en effet, les légionelles sont insensibles aux bêta-lactamines - qui sont les principaux antibiotiques prescrits en cas de pneumopathie - et il faut avoir recours à un traitement spécifique reposant sur les macrolides (érythromycine) ou les fluoroquinolones.

A l'heure actuelle, le diagnostic est réalisé par trois méthodes générales : la mise en évidence des bactéries à partir de prélèvements broncho-pulmonaires (soit directement par immunofluorescence soit par culture sur milieux sélectifs) ; la détection d'antigènes urinaires spécifiques ; la mise en évidence d'une augmentation significative du titre des anticorps dans le sérum (sérologie).

L'examen direct des prélèvements réalisé par microscopie à fluorescence se pratique, pour la majorité des réactifs commercialisés, à l'aide d'anticorps monoclonaux ou polyclonaux qui reconnaissent l'ensemble des sérogroupes connus de *L. pneumophila*. Le principal inconvénient de cette technique est sa faible sensibilité. Sa spécificité est également imparfaite (∼90%), du fait de réactions immunologiques croisées avec certaines bactéries comme *Pseudomonas aeruginosa, Bordetella pertusis* ou *Bacteroides fragilis*. La culture sur milieux spécifiques est la méthode de choix, notamment dans un but épidémiologique (typage des souches), mais donne des résultats tardifs puisque les légionelles cultivent en 3 à 4 jours, voire plus.

Les tests urinaires sont rapides et très spécifiques. Néanmoins, leur sensibilité est très variable, de 50-90% ; cette forte variabilité est liée non seulement aux caractéristiques du patient mais aussi au fait que les tests actuels permettent seulement la détection de *L. pneumophila sérogroupe 1*. Enfin, en présence d'une recherche positive d'antigènes urinaires de légionelles, il est indispensable de réaliser en parallèle une culture de prélèvements respiratoires pour l'isolement de la bactérie.

Le sérodiagnostic est tout particulièrement utile chez les patients qui ne produisent pas d'expectoration et en cas d'enquête épidémiologique. Il repose sur la mise en évidence d'une augmentation significative du taux des anticorps entre un sérum précoce et un sérum prélevé 4 à 6 semaines plus tard. Environ 30% des cas de légionellose sont identifiées par cette méthode à l'heure actuelle. Néanmoins, les antigènes utilisés dans les tests actuels sont constitués de simples extraits préparés directement à partir de culture de légionelles inactivées par la chaleur ou après ensemencement des bactéries dans le sac vitellin d'oeufs embryonnés. Les anticorps détectés reconnaissent en majorité des déterminants du lipopolysaccharide (LPS), qui peuvent être proches de déterminants du LPS d'autres bactéries et provoquer des réactions croisées. De fait, des réactions de ce type ont été décrites avec d'autres bactéries Gram négatives tels que *Pseudomonas sp*., *Bacteroides sp*., *Bordetella sp*. et certaines entérobactéries. Enfin, la plupart des tests actuels permettent seulement la détection des anticorps de type IgG, ce qui nécessite deux prises de sang à 4-6 semaines d'intervalle pour faire un diagnostic d'infection récente.

En résumé, les pratiques courantes ne répondent toujours pas aux attentes médicales pour établir le diagnostic précoce des légionelloses. En particulier, les tests sérologiques actuels reposent sur des méthodes de détection anciennes, non automatisables, ou utilisent des antigènes non caractérisés («soupes antigéniques»), dont la spécificité et la sensibilité sont peu satisfaisantes. La technique d'immunofluorescence indirecte (IFI) est la technique la plus employée pour le diagnostic sérologique (McDade JE, Shepard CC, Fraser DW, Tsai TR, Redus MA et Dowdle WR (1977), Legionnaires' disease : isolation of a bacterium and demonstration of its role in other respiratory disease, N Engl J Med. 297 (22) : 1197-203). Environ 70% des diagnostics au niveau européen sont réalisés par cette méthode. Il existe pourtant de très grandes variations selon les malades : ainsi pour un titre élevé précoce, la sensibilité est basse et la valeur prédictive est très faible (de l'ordre de 10-15% seulement) (Jarraud S, Reyrolle M et Etienne J. dans Freney J, Renaud F, Hansen W, Bollet C, Précis de bactériologie clinique, ed. ESKA, Paris, 2000).

Aucune technique de sérodiagnostic n'utilise donc actuellement d'antigènes purifiés spécifiques de *L. pneumophila*, et ne permet un diagnostic précoce de l'infection.

La protéine appelée 2A1, de séquence polypeptidique ID SEQ N°2, identifiée à partir du génome de *Legionella pneumophila*, est une protéine n'ayant ni homologue ni fonction connus.

Les inventeurs de la présente invention ont identifié de nouveaux polynucléotides et de nouveaux polypeptides dont les utilisations possibles sont décrites ci-après.

### Définitions

Les définitions suivantes sont données afin de faciliter la compréhension de certains termes utilisés dans cette description.

On entend par "polynucléotide", un polyribonucléotide ou un polydésoxyribonucléotide qui peut être un ADN ou un ARN modifié ou non.

Le terme polynucléotide inclut, sans limitation, un ADN simple brin ou double brin, un ADN composé d'un mélange d'une ou plusieurs région(s) simple brin et d'une ou plusieurs région(s) double brin, un ADN qui est un mélange de régions simple brin, double brin et/ou triple brin, un ARN simple brin ou double brin, un ARN composé d'un mélange d'une ou plusieurs région(s) simple brin et d'une ou plusieurs région(s) double brin et les molécules hybrides comprenant un ADN et un ARN qui peuvent comprendre des régions simple brin, double brin et/ou triple brin ou un mélange de régions simple brin et double brin. Le terme polynucléotide peut aussi comprendre un ARN et/ou un ADN comprenant une ou plusieurs régions triple brin. Les brins dans de telles régions peuvent provenir de la même molécule ou de molécules différentes. Par conséquent les ADN ou ARN, ayant des squelettes modifiés pour la stabilité ou d'autres raisons, sont inclus dans le terme polynucléotides. On entend également par polynucléotide, les ADNs et ARNs contenant une ou plusieurs bases modifiées. On entend par base modifiée, par exemple, les bases inhabituelles telles que l'inosine. Le terme polynucléotide vise également les polynucléotides de forme modifiée chimiquement, enzymatiquement ou métaboliquement. Les polynucléotides comprennent également les polynucléotides courts tels que les oligonucléotides.

On entend par "polypeptide", un peptide, un oligopeptide, un oligomère ou une protéine comprenant au moins deux acides aminés joints l'un à l'autre par une liaison peptidique normale ou modifiée.

Le terme polypeptide comprend les chaînes courtes, appelées peptides, oligopeptides et oligomères, et les chaînes longues, appelées protéines.

Un polypeptide peut être composé d'acides aminés autres que les 20 acides aminés codés par les gènes humains. Un polypeptide peut également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.

Un polypeptide peut être ramifié suite à une ubiquitination ou être cyclique avec ou sans ramification. Ce type de modifications peut être le résultat de processus de post-traduction naturel ou synthétique, qui sont bien connus de l'homme du métier.

On entend, par exemple, par modifications d'un polypeptide, l'acétylation, l'acylation, l'ADP-ribosylation, l'amidation, la fixation covalente de flavine, la fixation covalente d'un hème, la fixation covalente d'un nucléotide ou d'un dérivé nucléotidique, la fixation covalente d'un lipide ou d'un dérivé lipidique, la fixation covalente d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, l'oxydation, le processus protéolytique, la phosphorylation, la prénylation, la racémisation, la sénéloylation, la sulfatation, l'addition d'acides aminés, telle que l'arginylation ou l'ubiquitination. (PROTEINS STRUCTURE AND MOLECULAR PROPERTIES, 2nd Ed., T.E. Creighton, W.H. Freeman and Company, New York (1993) and Wold, F., Posttranslational Protein Modifications : Perspectives and Prospects, pgs 1-12 in POSTTRANSLATIONAL COVALENT MODIFICATION OF PROTEINS, B.C. Johnson, Ed., Academic Press, New York (1983) ; Seifter et al., Meth. Enzymol. 182 : 626-646 (1990) and Rattan et al., Protein Synthesis : Posttranslational Modifications and Aging, Ann. N.Y. Acad. Sci. 663 : 48-62 (1992)).

On entend par "isolé", modifié par la main de l'homme à partir de l'état naturel, c'est-à-dire que le polynucléotide ou le polypeptide présent dans la nature a été modifié ou isolé de son environnement naturel, ou les deux. Par exemple, un polynucléotide ou un polypeptide naturellement présent dans un organisme vivant n'est pas "isolé", mais le même polynucléotide ou polypeptide séparé des matériaux coexistants dans son état naturel est "isolé".

On entend par "pourcentage d'identité" entre deux séquences polynucléotidiques ou polypeptidiques le pourcentage de nucléotides ou d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons entre deux séquences polynucléotidiques ou polypeptidiques sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. Cette comparaison peut être réalisée grâce à un programme, par exemple le programme EMBOSS-Needle (alignement global Needleman-Wunsch) à l'aide de la matrice BLOSUM62/Open Gap 10.0 et Extension Penalty de 0,5 (Needleman, S.B. and Wunsch, C.D. (1970), J. Mol. Biol. 48, 443-453 et Kruskal, J.B. (1983), An overview of sequence comparison, In D. Sankoff and J.B. Kruskal, (ed), Time warps, strind edits and macromolecules : the theory and practice of sequence comparison, pp. 1-44 Addison Wesley).

Le pourcentage d'identité est calculé en déterminant le nombre de positions identiques pour lesquelles le nucléotide ou l'acide aminé est identique entre les deux séquences, en divisant ce nombre de positions identiques par le nombre total de positions dans la fenêtre de comparaison et en multipliant le résultat obtenu par 100.

Un polypeptide ayant, par exemple, une identité d'au moins 95% avec le polypeptide ID SEQ N°2 est un polypeptide comportant, au plus, 5 acides aminés modifiés sur 100 acides aminés, par rapport à ladite séquence. En d'autres termes jusqu'à 5% des acides aminés dans la séquence ID SEQ N°2 peuvent être délétés ou substitués par un autre acide aminé ou la séquence peut comporter jusqu'à 5% d'acides aminés en plus par rapport au nombre total d'acides aminés de la séquence ID SEQ N°2. Ces altérations de la séquence peuvent être situées aux positions amino et/ou carboxy-terminales de la séquence d'acides aminés ou à un endroit quelconque entre ces positions terminales, en un ou plusieurs emplacements. (Computational Molecular Biology, Lesk, A.M., ed., Oxford University Press, New York, 1988; Biocomputing: Informatics and Genome Projects, Smith, D.W., ed., Academic Press, New York, 1993 ; Computer Analysis of Sequence Data, Part I, Griffin, A.M., and Griffin, H.G., eds., Humana Press, New Jersey, 1994 ; Sequence Analysis in Molecular Biology, von Heinje, G., Academic Press, 1987).

Par analogie, un polynucléotide ayant une identité d'au moins 95% avec un second polynucléotide est donc un polynucléotide comportant, au plus, 5 nucléotides modifiés sur 100 nucléotides, par rapport à la séquence dudit second polynucléotide. En d'autres termes jusqu'à 5% des nucléotides dudit second polynucléotide peuvent être délétés ou substitués par un autre nucléotide, ou ledit polynucléotide peut comporter jusqu'à 5% de nucléotides en plus par rapport au nombre total de nucléotides du second polynucléotide. Ces modifications peuvent être positionnées aux extrémités 3' et/ou 5', ou à un endroit quelconque entre ces extrémités, en un seul ou plusieurs emplacements.

On entend par "cellule hôte", une cellule qui a été transformée ou transfectée, ou est capable de transformation ou de transfection, par une séquence polynucléotidique exogène.

On entend par "milieu de culture", le milieu dans lequel on purifie le polypeptide de l'invention. Ce milieu peut être constitué par le milieu extracellulaire et/ou le lysat cellulaire. Des techniques bien connues de l'homme du métier permettent également à ce dernier de redonner la conformation active au polypeptide, si la conformation dudit polypeptide a été altérée lors de l'isolation ou de la purification.

On entend par "fonction", l'activité biologique d'un polypeptide ou d'un polynucléotide.

La fonction d'un polypeptide conforme à l'invention est celle d'un antigène de Legionella pneumophila et celle du polynucléotide conforme à l'invention est de coder ce polypeptide.

On entend par "antigène", tout composé qui, seul ou en association avec un adjuvant ou porteur, est capable d'induire une réponse immunitaire spécifique. Cette définition comprend également tout composé présentant une analogie structurale avec ledit antigène capable d'induire une réponse immunologique dirigée contre ledit antigène.

On entend par "analogie structurale" une analogie aussi bien de la structure primaire (séquence) que de la structure secondaire (éléments structuraux), de la structure tertiaire (structure tridimensionnelle) ou de la structure quaternaire (association de plusieurs polypeptides dans un même complexe) (BIOCHEMISTRY, 4ème Ed, L. Stryer, New York, 1995).

On entend par "variant" d'un polynucléotide dit initial ou d'un polypeptide dit initial, respectivement, un polynucléotide ou un polypeptide qui en diffère par au moins un nucléotide ou un acide aminé, mais qui garde les mêmes propriétés intrinsèques, c'est-à-dire la même fonction.

Une différence dans la séquence polynucléotidique du variant peut altérer ou non la séquence d'acides aminés du polypeptide qu'il code, par rapport à un polypeptide initial. Néanmoins, par définition, ces variants doivent conférer la même fonction que la séquence polynucléotidique initiale, par exemple, coder un polypeptide ayant une fonction antigénique.

Le polynucléotide ou le polypeptide variant diffère généralement du polynucléotide initial ou du polypeptide initial par une (ou plusieurs) substitution(s), addition(s), délétion(s), fusion(s) ou troncation(s) ou plusieurs de ces modifications, prises en combinaison. Un variant non-naturel d'un polynucléotide initial ou d'un polypeptide initial peut être obtenu, par exemple, par mutagenèse dirigée ou par synthèse directe.

On définit comme "séquence polynucléotidique complémentaire à la séquence polynucléotidique", un polynucléotide qui peut être hybridé avec cette séquence polynucléotidique dans des conditions stringentes.

On entend généralement, mais pas nécessairement, par "conditions stringentes" les conditions chimiques qui permettent une hybridation lorsque les séquences polynucléotidiques ont une identité d'au moins 80%.

Ces conditions peuvent être obtenues selon les méthodes bien connues de l'homme du métier.

On entend par "anticorps", les anticorps monoclonaux, polyclonaux, chimériques, simple chaîne, humanisés, ainsi que les fragments Fab, incluant les produits d'un Fab ou d'une banque d'expression d'immunoglobulines.

Un anticorps immunospécifique peut être obtenu par administration à un animal d'un polypeptide donné, suivie d'une récupération des anticorps produits par ledit animal par extraction depuis ses fluides corporels. On peut également administrer à l'animal un variant dudit polypeptide, ou des cellules hôtes exprimant ce polypeptide.

Le terme "immunospécifique" appliqué au terme anticorps, vis-à-vis d'un polypeptide donné, signifie que l'anticorps possède une meilleure affinité pour ce polypeptide que pour d'autres polypeptides connus de l'art antérieur.

L'invention est définie par l'objet des revendications 1 à 13.

### Les polynucléotides

La présente invention a notamment pour objet un polynucléotide isolé tel que défini dans la revendication 1.

La présente invention concerne aussi un polynucléotide isolé, comprenant la séquence ID SEQ N°1, codant pour le polypeptide comprenant la séquence d'acides aminés ID SEQ N°2.

Les polynucléotides de l'invention peuvent être obtenus par les méthodes standard de synthèse d'ADN ou d'ARN.

Les polynucléotides conformes à l'invention peuvent également comprendre des séquences polynucléotidiques comme les séquences 5' et/ou 3' non-codantes, telles que, par exemple, des séquences transcrites, des séquences non-traduites, des séquences signal d'épissage, des séquences polyadénylées, des séquences de liaison avec des ribosomes ou encore des séquences qui stabilisent l'ARNm.

### Les vecteurs d'expression et les cellules hôtes

La présente invention a aussi pour objet un vecteur recombinant comprenant au moins un polynucléotide conforme à l'invention.

De nombreux systèmes d'expression peuvent être utilisés comme, par exemple, les chromosomes, les épisomes, les virus dérivés. Plus particulièrement, les vecteurs recombinants utilisés peuvent être dérivés de plasmides bactériens, de transposons, d'épisome de levure, d'éléments d'insertion, d'éléments chromosomiques de levures, de virus tels que les baculovirus, les papillonna virus comme SV40, les vaccinia virus, les adénovirus, les fox pox virus, les pseudorabies virus, les rétrovirus.

Ces vecteurs recombinants peuvent également être des dérivés de cosmides ou de phagemides. La séquence polynucléotidique peut être insérée dans le vecteur recombinant d'expression par les méthodes bien connues de l'homme du métier.

Le vecteur recombinant peut comprendre des séquences polynucléotidiques de contrôle de la régulation de l'expression du polynucléotide ainsi que des séquences polynucléotidiques permettant l'expression et la transcription d'un polynucléotide de l'invention et la traduction d'un polypeptide de l'invention, ces séquences étant choisies en fonction des cellules hôtes mises en oeuvre.

La présente invention a aussi pour objet une cellule hôte comprenant un vecteur recombinant conforme à l'invention.

L'introduction du vecteur recombinant dans une cellule hôte peut être effectuée selon les méthodes bien connues de l'homme du métier telles que la transfection par phosphate de calcium, la transfection par lipides cationiques, l'électroporation, la transduction ou l'infection.

Les cellules hôtes peuvent être, par exemple, des cellules bactériennes telles que des cellules de streptocoques, de staphylocoques, d'*Escherichia coli* ou de *Bacillus subtilis,* des cellules de champignons telles que des cellules de levure et des cellules d'*Aspergillus*, des cellules de *Streptomyces*, des cellules d'insectes telles que des cellules de *Drosophilia S2* et de *Spodoptera Sf9,* des cellules animales, telles que les cellules CHO, COS, HeLa, C127, BHK, HEK 293 ou encore des cellules végétales.

### Les polypeptides

La présente invention a aussi pour objet un polypeptide tel que défini dans la revendication 6.

On décrit également ici un procédé de préparation d'un polypeptide tel que défini ci-dessus, dans lequel une cellule hôte définie précédemment est cultivée et ledit polypeptide est isolé du milieu de culture.

Le polypeptide peut être purifié à partir des cellules hôtes, selon les méthodes bien connues de l'homme du métier telles que la précipitation à l'aide d'agents chaotropiques comme les sels, en particulier le sulfate d'ammonium, l'éthanol, l'acétone ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Les anticorps

La présente invention a aussi pour objet les anticorps immunospécifiques pour les polypeptides conformes à l'invention, tels que définis précédemment.

Les anticorps immunospécifiques peuvent être obtenus par administration d'un polypeptide selon l'invention, d'un de ses fragments, d'un analogue ou d'un fragment épitopique ou d'une cellule exprimant ce polypeptide, à un mammifère, de préférence non humain, selon les méthodes bien connues de l'homme du métier.

Pour la préparation d'anticorps monoclonaux, on peut utiliser des méthodes usuelles de production d'anticorps, à partir de lignées cellulaires, telles que la technique des hybridomes, la technique des triomes, la technique des hybridomes de cellules B humaines et la technique des hybridomes EBV.

### Sérologie

La présente invention a également pour objet l'utilisation d'un polypeptide selon l'invention pour détecter, *in vitro*, dans des échantillons biologiques la présence d'anticorps dirigés contre *Legionella pneumophila*.

L'invention vise également l'utilisation d'anticorps, selon l'invention, pour détecter, *in vitro*, dans des échantillons biologiques la présence d'antigènes de *Legionella pneumophila*.

On décrit, en outre, l'utilisation de polypeptides et d'anticorps selon l'invention, pour détecter, périodiquement, *in vitro*, respectivement, les anticorps dirigés contre *Legionella pneumophila* et les antigènes de *Legionella pneumophila* et ainsi suivre l'évolution de la pathologie et de l'effet d'un traitement appliqué à un patient.

Les échantillons biologiques testés peuvent être du sang, de l'urine, de la salive, du liquide de ponction de sérologie (par exemple liquide céphalo-rachidien, liquide pleural ou liquide articulaire) ou l'un de leurs constituants (par exemple, le sérum).

### Les kits

L'invention a également pour objet des kits de diagnostic *in vitro* comprenant au moins l'un des polypeptides conformes à l'invention ainsi que des kits de diagnostic *in vitro* comprenant au moins l'un des anticorps conformes à l'invention.

### Les vaccins

La présente invention concerne également une composition pharmaceutique, utilisable comme vaccin, renfermant à titre de principe actif au moins un polypeptide selon l'invention ou un polynucléotide ou un vecteur recombinant ou une cellule hôte selon l'invention.

### Partie expérimentale

### A) Protocole d'obtention des antigènes

### Clonage de la séquence codant la protéine 2A1

Le gène codant pour les séquences de la protéine 2A1, qui est un antigène, est obtenu par amplification par PCR à partir de l'ADN génomique de la bactérie *Legionella pneumophila* (souche Philadelphia-1, ATCC 33152) en utilisant comme couple d'amorces :
- l'oligonucléotide sens contenant la séquence :
   5'-ACGTTTGTTTTAAAGGAATTTG-3' ; et
- l'oligonucléotide antisens contenant la séquence :
   5'-TACGGTCGGTTTGCTGGTC-3'

Le fragment ainsi amplifié correspondant est cloné dans un vecteur selon des techniques classiques bien connues de l'homme du métier. Ce vecteur permet la production de la protéine clonée sous contrôle d'un promoteur inductible par l'isopropyl-thiogalactoside (IPTG). La protéine clonée correspond à la séquence d'acides aminés ID SEQ N°2.

### Expression de la protéine 2A1

Une souche d'Escherichia coli est transformée par le vecteur d'expression précédemment décrit. Les bactéries sélectionnées sont mises à cultiver une nuit à 30°C sous agitation dans 30 ml de milieu Luria Bertani (LB, J. Miller, "A short Course in Bacterial Genetics", Cold Spring Harbor Laboratory Press, 1992) contenant de l'ampicilline à une concentration finale de 100 µg/ml. Le lendemain, la culture est diluée au 1/50ème dans un volume final de 1 litre de milieu LB additionné d'ampicilline à une concentration finale de 100 µg/ml et incubée à 30°C sous agitation. Lorsque la turbidité de la culture atteint une valeur d'absorbance à 600 nm (A600) d'environ 0,7, la production de la protéine est induite par l'IPTG à une concentration finale de 0,1 mM. Les bactéries sont récoltées par centrifugation (10 minutes à 1400xg et à 4°C) lorsque la turbidité de la culture atteint une A600 d'environ 1,5.

### Purification

Après centrifugation, les cellules sont remises en suspension dans un tampon Tris-HCl 20 mM à pH 8,0 contenant du saccharose à 0,5 mM, puis traitées par du lysozyme (0,2 g/l en présence d'acide éthylènediaminotétraacétique (EDTA) 12,5 mM, de DNase, RNase et PMSF (fluorure de phénylméthylsulfonyle). La suspension est incubée 30 minutes à 4°C puis centrifugée 10 minutes à 4°C à 15500xg. Le culot est congelé à -20°C pendant au moins une nuit.

Après décongélation, les bactéries sont reprises dans un tampon Mes 25 mM à pH 6,0, puis soniquées 4 fois 20 secondes dans la glace. Après centrifugation à 15500xg à 4°C pendant 30 minutes, le surnageant est filtré sur membrane de porosité 0,22 µm. Le filtrat est alors déposé sur une colonne échangeuse de cation (par exemple, SP-Sépharose 12 ml, Amersham Biosciences). Après lavage de la colonne, la protéine est éluée par un gradient linéaire de 0 à 1 M de NaCl dans du tampon Mes [acide 2-(N-morpholino) éthane sulfonique] 25 mM à pH 6,0 en 20 volumes de colonne. Les fractions contenant la protéine sont rassemblées et les protéines sont précipitées par du sulfate d'ammonium à une concentration finale de 0,6 g/l. La solution est laissée au moins une nuit à 4°C puis centrifugée 30 minutes à 20800xg. Le culot est alors repris dans un volume le plus faible possible (en général 300 µl de tampon Na₂HPO₄/NaH₂PO₄ 50 mM pH 8,0 contenant du NaCl 100 mM puis déposé sur une colonne de gel filtration, par exemple SuperdexHR75-10/30, Amersham). Les fractions éluées contenant la protéine sont rassemblées et du glycérol est ajouté à une concentration finale de 20%. Les protéines purifiées sont alors stockées à -20°C jusqu'à leur utilisation dans les tests.

Les concentrations des protéines sont déterminées spectrophotométriquement à partir des coefficients d'absorption calculés par la méthode de Pace (Pace CN, Vajdos F., Fee L., Grimsley G. Et Gray T., (1995), Protein Science 4, 2411-2423). La pureté des protéines est vérifiée par analyse par élecrophorèse SDS-PAGE et par spectrométrie de masse.

### B) Test de diagnostic in vitro

Il a été utilisé des sérums provenant de patients ayant eu une infection documentée à *Legionella pneumophila* (collection du laboratoire). L'infection a pu être avérée soit par l'isolement/culture des bactéries à partir de prélèvements broncho-pulmonaires, soit par la mise en évidence d'une séroconversion ou encore par un test urinaire positif.

Les sérums témoins correspondaient à des sérums de donneurs de sang (collection du laboratoire).

La fixation, sur la protéine 2A1 recombinante purifiée (obtenue comme décrit précédemment), des anticorps présents dans les sérums peut être évaluée soit par des tests en Western Blot soit par la technique ELISA.

### Protocole du test en Western blot

Après transfert, sur une membrane de nitrocellulose, de la protéine purifiée 2A1, la membrane est saturée 30 minutes à l'aide d'une solution de "tampon salin phosphate" (PBS) contenant 3% de lait demi-écrémé. Après trois lavages par du PBS contenant du polyoxyéthylène sorbitan (Tween) à 0,05%, la membrane est mise en présence du sérum testé à la dilution adéquate (soit 1/300ème) dans du tampon PBS contenant 3% de lait demi-écrémé pendant 45 minutes. Après trois nouveaux lavages, des anticorps anti-immunoglobulines G, A, M humaines de chèvre (par exemple 170-6462, Biorad), marqués à la phosphatase alcaline, sont ajoutés simultanément, pendant une période de 30 minutes après avoir été dilués selon le protocole du fournisseur dans du tampon PBS contenant 3% de lait demi-écrémé. Trois nouveaux lavages sont réalisés puis du phosphate de 5-bromo-4-chloro-3-indolyle et du nitroblue tetrazolium sont ajoutés selon les indications du fournisseur jusqu'à l'obtention du résultat. Un résultat "positif" correspond à une précipitation du substrat sur la membrane à la position de 2A1.

### Protocole du test en ELISA

Les plaques ELISA sont laissées pendant une nuit à 4°C en présence de 0,5 µg d'antigène purifié (protéine recombinante 2A1) dans du "tampon salin phosphate" (PBS). Après quatre lavages par du PBS contenant du polyoxyéthylène sorbitan (Tween) à 0,05%, les plaques sont saturées une heure à 37°C par du PBS-Tween contenant 5% de lait demi-écrémé (250 µl par puits). Quatre nouveaux lavages sont réalisés, puis 100 µl de chaque sérum positif, à la dilution adéquate (soit 1/300ème) dans du tampon PBS-Tween contenant 5% de lait demi-écrémé, sont ajoutés dans chaque puits. La plaque est ensuite laissée à 25°C pendant 30 minutes. Après quatre nouveaux lavages, des anticorps anti-immunoglobulines G, A, M humaines de chèvre marqués à la phosphatase alcaline sont ajoutés simultanément pendant 30 minutes à 25°C après avoir été dilués selon le protocole du fournisseur dans du tampon PBS-Tween contenant 5% de lait demi-écrémé. Quatre nouveaux lavages sont réalisés puis 100 µl de substrat (phosphate de p-nitrophényle), pNPP, par exemple A-3469, Sigma) sont ajoutés. L'absorbance à 405 nm de chacun des puits est mesurée après une incubation de 30 minutes à 37°C.

### Résultats et interprétation

Un résultat type obtenu est présenté dans le tableau ci-après, sachant que les sérums dits "positifs" dans ce tableau sont ceux contenant des anticorps contre *Legionella pneumophila* identifiés par leur liaison avec les polypeptides (antigènes) de l'invention par Western blot :

**Tableau de résultats du test en Western blot**

| | |
|---|---|
| Nombre de sérums de malades infectés par *Legionella pneumophila,* diagnostiqués selon l'art antérieur et soumis au diagnostic selon l'invention | 24 |
| Sérums "positifs" parmi les 24 testés | 18, soit 75% |
| | |
| Sérums de donneurs de sang comme témoins | 111 |
| Séums "positifs" parmi les 111 testés | 4, soit 3,6% |

Des résultats similaires sont obtenus en ELISA.

D'après le Tableau, on constate que, par le test Western blot ou par le test ELISA, on identifie *in vitro* au moins 70 % des infections à *Legionella pneumophila*, grâce à l'antigène selon l'invention. Il est donc démontré, d'une part, l'existence chez l'homme d'une réponse anticorps significative (la probabilité associée à un test de χ² est inférieure à 0,05) vis-à-vis de la protéine 2A1 au cours des infections à *Legionella pneumophila* et, d'autre part, que la protéine 2A1 est pertinente pour le diagnostic sérologique de ce type d'infection.

De plus l'utilisation de l'antigène 2A1 selon l'invention permet, dans certains cas, l'identification de malades non détectés à ce stade de l'infection (mais confirmés dans des analyses ultérieures) par les techniques de sérologie de l'art antérieur.

## Revendications

1. Polynucléotide isolé comprenant :
a) la séquence polynucléotidique SEQ ID NO : 1, ou
b) une partie de la séquence polynucléotidique SEQ ID NO : 1, ou
c) une séquence polynucléotidique ayant au moins 60% d'identité avec la séquence polynucléotidique SEQ ID NO : 1 ou avec la partie de séquence telle que définie sous b), ou
d) une séquence polynucléotidique complémentaire à une séquence polynucléotidique telle que définie sous a), b) ou c),
dans lequel les polynucléotides définis sous b) et c) ont la même fonction de coder un antigène de *Legionella pneumophila* que la séquence SEQ ID NO : 1 et codent un polypeptide ayant la capacité de détecter, *in vitro,* dans des échantillons biologiques, la présence d'anticorps dirigés contre *Legionella pneumophila*.

2. Polynucléotide isolé selon le point a) de la revendication 1, **caractérisé en ce qu'**il code pour le polypeptide comprenant la séquence d'acides aminés SEQ ID NO : 2.

3. Vecteur recombinant comprenant un polynucléotide selon la revendication 1 ou 2.

4. Cellule hôte comprenant un vecteur recombinant selon la revendication 3.

5. Procédé de préparation d'un polypeptide, **caractérisé en ce qu'**il consiste à cultiver une cellule hôte selon la revendication 4 et à isoler ledit polypeptide du milieu de culture.

6. Polypeptide isolé comprenant:
a) la séquence d'acides aminés SEQ ID NO : 2, ou
b) une partie de la séquence d'acides aminés SEQ ID NO : 2, ou
c) une séquence d'acides aminés ayant au moins 60% d'identité avec la séquence d'acides aminés SEQ ID NO : 2 ou avec la partie de séquence telle que définie sous b),
dans lequel les polypeptides définis sous b) et c) ont la même fonction d'antigène de *Legionella pneumophila* que la séquence d'acides aminés SEQ ID NO : 2 et ont la capacité de détecter, *in vitro*, dans des échantillons biologiques, la présence d'anticorps dirigés contre *Legionella pneumophila*.

7. Anticorps immunospécifique pour un polypeptide selon la revendication 6.

8. Utilisation du polypeptide selon la revendication 6 pour détecter, *in vitro*, la présence d'anticorps à *Legionella pneumophila* dans des échantillons biologiques.

9. Utilisation de l'anticorps selon la revendication 7 pour détecter, *in vitro,* la présence d'antigènes à *Legionella pneumophila* dans des échantillons biologiques.

10. Composition pharmaceutique, notamment vaccin, comprenant à titre de principe actif au moins un polypeptide selon la revendication 6, ainsi qu'un excipient pharmaceutiquement acceptable.

11. Composition pharmaceutique, notamment vaccin, comprenant au moins un polynucléotide selon l'une des revendications 1 ou 2, ou un vecteur recombinant selon la revendication 3 ou une cellule hôte selon la revendication 4.

12. Kit de diagnostic pour détecter, *in vitro*, dans des échantillons biologiques la présence d'anticorps produits à la suite d'une infection à *Legionella pneumophila*, **caractérisé en ce qu'**il comprend au moins un polypeptide tel qu'identifié dans la revendication 6.

13. Kit de diagnostic pour détecter, *in vitro*, dans des échantillons biologiques la présence d'antigène issu d'une infection à *Legionella pneumophila*, **caractérisé en ce qu'**il comprend au moins un anticorps tel qu'identifié dans la revendication 7.

## Claims

1. Isolated polynucleotide comprising:
a) the polynucleotide sequence SEQ ID NO: 1, or
b) a part of the polynucleotide sequence SEQ ID NO:1, or
c) a polynucleotide sequence having at least 60% identity with the polynucleotide sequence SEQ ID NO: 1 or with the part of the sequence as defined under b), or
d) a polynucleotide sequence complementary to a polynucleotide sequence as defined under a), b) or c),
in which the polynucleotides defined under b) and c) have the same function of coding an antigen of *Legionella pneumophila* as the sequence SEQ ID NO: 1 and coding a polypeptide having the ability to detect, *in vitro*, in biological samples, the presence of antibodies against *Legionella pneumophila*.

2. Isolated polynucleotide according to point a) of claim 1, **characterised in that** it codes for the polypeptide comprising the amino acid sequence SEQ ID NO: 2.

3. Recombinant vector comprising a polynucleotide according to claim 1 or claim 2.

4. Host cell comprising a recombinant vector according to claim 3.

5. Method of preparing a polypeptide, **characterised in that** it consists of cultivating a host cell according to claim 4, and of isolating the said polypeptide from the culture medium.

6. Isolated polypeptide comprising:
a) the amino acid sequence SEQ ID NO: 2, or
b) a part of the amino acid sequence SEQ ID NO: 2, or
c) an amino acid sequence having at least 60% identity with the amino acid sequence SEQ ID NO: 2 or with the part of the sequence as defined under b),
in which the polypeptides defined under b) and c) have the same function of an antigen to *Legionella pneumophila* as the amino acid sequence SEQ ID NO: 2 and have the ability to detect, *in vitro,* in biological samples, the presence of antibodies against *Legionella pneumophila*.

7. Immunospecific antibody for a polypeptide according to claim 6.

8. Use of a polypeptide according to claim 6 to detect, *in vitro*, the presence of antibodies of *Legionella pneumophila* in biological samples.

9. Use of the antibodies according to claim 7 to detect, *in vitro,* the presence of antigens of *Legionella pneumophila* in biological samples.

10. Pharmaceutical composition, especially a vaccine, comprising as an active principal at least one polypeptide according to claim 6, as well as a pharmaceutically acceptable excipient.

11. Pharmaceutical composition, especially a vaccine, comprising at least one polynucleotide according to claim 1 or claim 2, or a recombinant vector according to claim 3 or a host cell according to claim 4.

12. Diagnostic kit for detecting, *in vitro,* in biological samples, the presence of antibodies produced as a result of an infection of *Legionella pneumophila* **characterised in that** it comprises at least one polypeptide such as identified in claim 6.

13. Diagnostic kit for detecting, *in vitro*, in biological samples, the presence of an antigen arising from an infection of *Legionella pneumophila*, **characterised in that** it comprises at least one antibody such as identified in claim 7.

## Patentansprüche

1. Isoliertes Polynucleotid umfassend:
a) die Polynucleotidsequenz SEQ ID Nr. 1 oder
b) einen Teil der Polynucleotidsequenz SEQ ID Nr. 1 oder
c) eine Polynucleotidsequenz, die mindestens 60 % Identität mit der Polynucleotidsequenz SEQ ID Nr. 1 oder mit der unter b) definierten Teilsequenz aufweist, oder
d) eine zu einer unter a), b) oder c) definierten Polynucleotidsequenz komplementäre Polynucleotidsequenz,
wobei die unter b) und c) definierten Polynucleotide dieselbe Funktion des Kodierens für ein Antigen von *Legionella pneumophila* wie die Sequenz SEQ ID Nr. 1 haben und für ein Polypeptid kodieren, das die Fähigkeit zum Nachweisen in vitro des Vorhandenseins gegen *Legionella pneumophila* gerichteter Antikörper in biologischen Proben besitzt.

2. Isoliertes Polynucleotid gemäß a) des Anspruchs 1, **dadurch gekennzeichnet, dass** es für das die Aminosäuresequenz SEQ ID Nr. 2 umfassende Polypeptid kodiert.

3. Rekombinanter Vektor umfassend ein Polynucleotid gemäß Anspruch 1 oder 2.

4. Wirtszelle umfassend einen rekombinanten Faktor gemäß Anspruch 3.

5. Verfahren zur Herstellung eines Polypeptids, **dadurch gekennzeichnet, dass** es aus dem Kultivieren einer Wirtszelle gemäß Anspruch 4 und dem Isolieren des Polypeptids aus dem Kulturmedium besteht.

6. Isoliertes Polypeptid umfassend:
a) die Aminosäuresequenz SEQ ID Nr. 2 oder
b) einen Teil der Aminosäuresequenz SEQ ID Nr. 2 oder
c) eine Aminosäuresequenz, die mindestens 60 % Identität mit der Aminosäuresequenz SEQ ID Nr. 2 oder mit der unter b) definierten Teilsequenz aufweist,
wobei die unter b) und c) definierten Polypeptide dieselbe Funktion eines Antigens von *Legionella pneumophila* wie die Aminosäuresequenz SEQ ID Nr. 2 haben und die Fähigkeit zum Nachweisen in vitro des Vorhandenseins gegen *Legionella pneumophila* gerichteter Antikörper in biologischen Proben besitzen.

7. Für ein Polypeptid gemäß Anspruch 6 immunspezifischer Antikörper.

8. Verwendung des Polypeptids gemäß Anspruch 6 zum Nachweisen in vitro des Vorhandenseins von Antikörpern auf *Legionella pneumophila* in biologischen Proben.

9. Verwendung von Antikörpern gemäß Anspruch 7 zum Nachweisen in vitro des Vorhandenseins von Antigenen auf *Legionella pneumophila* in biologischen Proben.

10. Pharmazeutische Zusammensetzung, insbesondere Vakzin, umfassend als aktives Prinzip wenigstens ein Polypeptid gemäß Anspruch 6 sowie einen pharmazeutisch annehmbaren Arzneimittelhilfsstoff.

11. Pharmazeutische Zusammensetzung, insbesondere Vakzin, umfassend wenigstens ein Polynucleotid gemäß einem der Ansprüche 1 oder 2 oder einen rekombinanten Vektor gemäß Anspruch 3 oder eine Wirtszelle gemäß Anspruch 4.

12. Diagnostischer Kit zum Nachweisen in vitro des Vorhandenseins infolge einer Infektion mit *Legionella pneumophila* gebildeter Antikörper in biologischen Proben, **dadurch gekennzeichnet, dass** er wenigstens ein in Anspruch 6 bezeichnetes Polypeptid umfasst.

13. Diagnostischer Kit zum Nachweisen in vitro des Vorhandenseins eines aus einer Infektion mit *Legionella pneumophila* stammenden Antigens in biologischen Proben, **dadurch gekennzeichnet, dass** er wenigstens einen in Anspruch 7 bezeichneten Antikörper umfasst.
